# EUROPÄISCHE PATENTSCHRIFT

(11) **EP 0 839 543 B1**
(45) Veröffentlichungstag und Bekanntmachung des Hinweises auf die Patenterteilung: **12.11.2003**
(21) Anmeldenummer: 97113345.9
(22) Anmeldetag: 01.08.1997
(51) Int. Cl.: A61M 1/16, A61L 2/18, C02F 1/70

(54) **Verfahren und Vorrichtung zur Desinfektion eines Dialysegerätes**
Method and means for desinfecting a dialysis apparatus
Méthode et dispositif de désinfection d'un appareil d'hémodyalise

(30) Priorität: 02.10.1996 DE 19640839
(43) Veröffentlichungstag der Anmeldung: 06.05.1998
(73) Patentinhaber: Fresenius Medical Care Deutschland GmbH, 61350 Bad Homburg v.d.H. (DE)
(72) Erfinder: Spickermann, Reiner, 97535 Wasserlosen-Burghausen (DE)
(74) Vertreter: Laufhütte, Dieter, Dr.-Ing.

(56) Entgegenhaltungen:
- EP-A- 0 364 367
- US-A- 5 292 488

## Beschreibung

Die Erfindung betrifft ein Verfahren und eine Vorrichtung zur Desinfektion eines Dialysegerätes.

Medizinische Geräte, wie etwa Dialysegeräte, müssen in regelmäßigen Abständen desinfiziert bzw. in einen möglichst sterilen Zustand gebracht werden.

### Dies sei am Beispiel eines Dialysegeräts verdeutlicht:

Das Blut eines Patienten fließt über einen extrakorporalen Blutkreislauf zur Reinigung zu einem Dialysegerät, in welchem die notwendigen Reinigungsschritte durchgeführt werden.

Da das Dialysat über die Dialysemembran mit dem Blut in indirekten Kontakt kommt, muß der Dialysatkreislauf möglichst steril gehalten werden. Eventuell vorhandene Toxine könnten über die Membran sonst in das Patientenblut gelangen.

Aus der EP 0 364 367 ist ein Verfahren zur Desinfektion eines Dialysegerätes bekannt.

Aus der US 5,292,488 ist eine Vorrichtung zur Desinfektion von Kontaktlinsen bekannt. Die Vorrichtung enthält eine Aufnahme für die zu desinfizierenden Kontaktlinsen sowie für ein katalytisch wirksames Mittel, mittels dessen das aus H₂O₂ bestehende Desinfektionsmittel zersetzt wird. Die Desinfektion sowie die Zersetzung des Desinfektionsmittels beginnen mit Einführung der die Kontaktlinsen sowie das katalytisch wirksame Mittel enthaltenden Aufnahme in die entsprechende Desinfektionslösung.

Der weltweit am häufigsten zur Desinfektion von Dialysegeräten eingesetzte Wirkstoff ist NaOCI (Bleach). Bei diesem hochwirksamen Desinfektions- und Bleichmittel ist jedoch die Umweltbelastung sehr groß. Hier sollte insbesondere die Abwasserproblematik erwähnt werden. Andererseits weist NaOCI jedoch Vorteile bezüglich der einfachen Handhabung und der effektiven Reinigung/Desinfektion auf. Ferner ist NaOCI in zahlreichen Staaten oftmals das einzig verfügbare Desinfektionsmittel und zudem relativ preisgünstig.

Aufgabe der Erfindung ist es daher, ein Verfahren und eine Vorrichtung zur Desinfektion von Dialysegeräten zur Verfügung zu stellen, bei denen die Verwendung von Desinfektionsmittel ohne Gefahren für die Umwelt möglich ist.

Diese Aufgabe wird gelöst durch ein Verfahren gemäß dem Patentanspruch 1 bzw. eine Vorrichtung gemäß dem Patentanspruch 9.

Mit dem erfindungsgemäßen Verfahren bzw. der erfindungsgemäßen Vorrichtung ist es nun erstmals möglich, Dialysegeräte unter Verwendung von z.B. NaOCI oder 03 zu desinfizieren, ohne daß es zu einer Belastung bzw. zu Gefahren für die Umwelt kommt.

Gemäß einer vorteilhaften Ausgestaltung des erfindungsgemäßen Verfahrens bzw. der erfindungsgemäßen Vorrichtung sind ferner Mittel zur UV-Photooxidation von RO-Wasser vorgesehen, wodurch ultrareines Wasser herstellbar ist. Das derart hergestellte ultrareine Wasser kann beispielsweise zum Nachspülen der Vorrichtung nach einer Desinfektion, oder aber auch bei Herstellung von ultrareiner Dialysierflüssigkeit aus Dialysierflüssigkeitkonzentrat verwendet werden.

Das durch die katalytische Zersetzung erzeugte NaCI kann wenigstens teilweise mittels einer Elektrolysereaktion wieder in NaOCI umgewandelt werden. Dieses NaOCl kann dem Desinfektionskreislauf wieder zugeführt werden.

Gemäß einer weiteren vorteilhaften Ausgestaltung wird der Desinfektionsmittelgehalt der verwendeten Desinfektionsflüssigkeit bestimmt. So kann festgestellt werden, ob der Desinfektionsmittelgehalt in der Desinfektionsflüssigkeit eine ausreichende Desinfektion des Gerätes gewährleistet. Ferner muß vor dem Anschluß eines Patienten an eine Dialysevorrichtung gewährleistet sein, daß sämtliches Desinfektionsmittel aus dem Gerät ausgespült ist.

Die Bestimmung des Desinfektionsmittelgehaltes erfolgt vorzugsweise unmittelbar während und/oder nach der katalytischen Zersetzung des NaOCI.

Vorteilhaft erfolgt die Bestimmung des Desinfektionsmittelgehaltes mittels einer Redox-Elektrode. Diese Methode erweist sich als sehr einfach und zuverlässig, und kann ohne weiteres in ein Dialysegerät integriert werden, womit eine weitere Automatisierung des Dialyseverfahrens möglich ist.

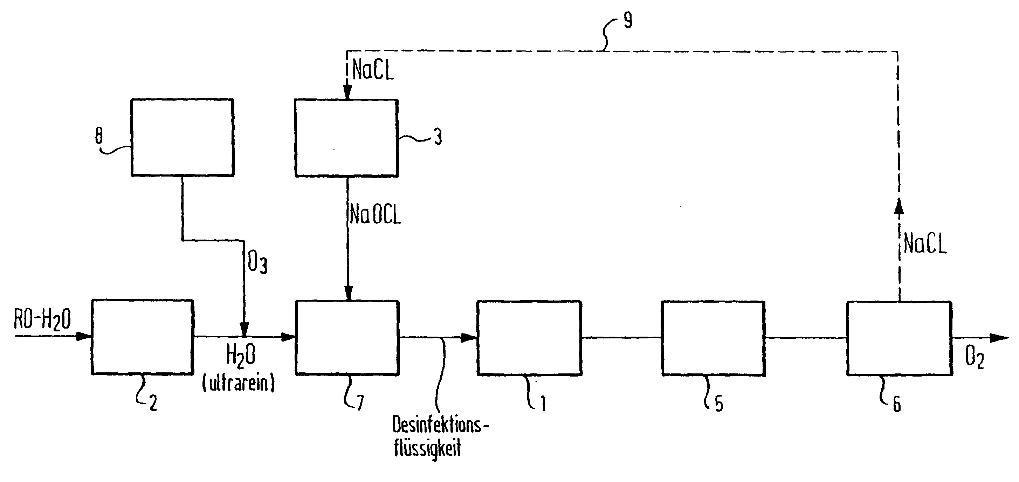

Ein Ausführungsbeispiel der Erfindung wird nachstehend anhand der Zeichnung näher erläutert, in deren einziger Figur schematisch die erfindungsgemäßen Verfahrensschritte dargestellt sind. Es sei darauf hingewiesen, daß die dargestellten Verfahrensschritte durch entsprechende Mittel innerhalb des Dialysegerätes ausgeführt werden können. Es ist jedoch gleichfalls möglich, ein herkömmliches Dialysegerät extern mit derartigen Mitteln zu verbinden.

Blut von einem Patienten wird einer zentralen Dialyseeinheit 1 zugeführt und in dieser Dialyseeinheit 1 gereinigt bzw. dialysiert. Dann wird das Blut dem Patienten wieder zugeführt. Die vorliegende Erfindung behandelt eine Desinfektion der Leitungen bzw. Abschnitte der Dialyseeinheit, durch welche Dialysat fließt. Besteht die Blutseite aus Einwegteilen, wird in der Regel eine Desinfektion nicht notwendig sein.

RO-Wasser wird einer UV-Photooxidationskammer 2 zugeführt. Das Wasser wird dort einer UV-Strahlung, beispielsweise der UV-Strahlung einer Quecksilber-Niederdrucklampe, ausgesetzt. UV-Licht im Spektralbereich von 200 - 300 Nanometern tötet Mikroorganismen, indem es die DNA der Zellen zerstört. Quecksilber-Niederdrucklampen erzeugen die größte Energie bei einer Wellenlänge von 254 Nanometern. Dieser Wert liegt ganz in der Nähe der optimalen Wellenlänge zur Entkeimung, welche 260 Nanometer beträgt.

Speziell entwickelte Lampen, für die nur hochreines Quarz verwendet wird, ermöglichen ferner die Abstrahlung einer Wellenlänge bei 185 Nm. Der kombinierte Effekt, der durch UV-Licht mit den Wellenlängen 185 Nm und 254 Nm entsteht, bewirkt die Photooxidation von gelösten organischen Verbindungen.

Die Emission von UV-Licht 185 Nm katalysiert ferner die Reaktion von im Wasser gelösten Sauerstoff zu Ozon.

Insgesamt läßt sich durch die UV-Photooxidation des RO-Wasser ultrareines Wasser herstellen. Bei Bedarf kann diesem ultrareinen Wasser weiteres Ozon zugegeben werden, wie dies schematisch in der Zeichnung dargestellt ist.

Das zugegebene Ozon kann mittels eines Ozongenerators, beispielsweise über eine Bleidioxidelektrode oder mittels eines Siemens-
Generators 8, hergestellt werden. Die Benutzung des Verfahrens der elektrolytischen Oxidation mit Bleidioxid-Anoden bietet insofern Vorteile gegenüber anderen Erzeugungsverfahren, als keine Gasbildung erfolgt und hohe Konzentrationen erzielbar sind. Selbstverständlich sind auch andere Verfahren zur Erzeugung von Ozon denkbar. Ferner ist die Zuführung bereits hergestellten Ozons möglich.

In einem Generator 3 erfolgt die elektrolytische Herstellung von NaOCI. Beim bevorzugten Herstellungsverfahren wird 10%ige Natriumchloridlösung derart elektrolysiert, daß das am Pluspol freiwerdende Chlor und das am Minuspol entstehende NaOH miteinander reagieren nach der Gleichung Cl2 + 2NaOH = NaOCl + NaCI + H20.

Es ist ebenfalls denkbar, die obengenannte elektrolytische Reaktion von NaCl im vorher erzeugten ultrareinen Wasser ablaufen zu lassen, so daß auf eine besondere Zuführung des NaOCI zum ultrareinen Wasser verzichtet werden kann. Das so erzeugte Desinfektionsmittel wird über eine Zuführeinrichtung 7 der zentralen Dialyseeinheit 1 zugeführt.

Ozon weist insofern Vorteile auf, als es zu einer weniger starken Beeinträchtigung einer Dialysemembran führt, falls der Filter der Dyalisemaschine verbleibt oder zusätzliche Filter wie Diasafe oder online Filter in der Maschine sind.

Es ist ebenfalls denkbar, für besondere Anwendungsfälle die Desinfektion eines medizinischen Gerätes ausschließlich mittels Ozon zu gewährleisten.

Ozon ist auch vorteilhaft bei der keimfreien Konservierung von Wasser über mehrere Tage verwendbar. Insbesondere ein Dialysegerät (Heimdialysegerät) wird nur alle zwei bis drei Tage benutzt. Ein ausreichender Ozongehalt des im Gerät verbleibenden Wassers verhindert das Keimwachstum bis zur nächsten Benutzung.

Zur Gewährleistung einer einwandfreien Desinfektion ist eine Redox-Elektrode 5 vorgesehen, mittels derer das Redox-Potential der Desinfektionsflüssigkeit bestimmbar ist. Diese Redox-Elektrode dient zum Nachweis der Anwesenheit von Desinfektionsmittel während des Desinfektionsvorgangs, um so einen korrekten Ablauf des Desinfektionsprogramms sicherzustellen. Sie dient ferner zum Nachweis der Abwesenheit von Desinfektionsmittel zur Kontrolle eines korrekten Freispülens der zentralen Dialyseeinheit 1.

Eine solche Redox-Elektrode läßt sich mit geringem Platzaufwand in ein Dialysegerät einbauen. Der zur Zeit noch manuell durchzuführende Nachweis des Freispülens, beispielsweise mittels Teststreifen (KJ-Stärkepapier, Peroxidtest, etc.) kann entfallen.

Durch das Vorsehen einer Redox-Elektrode ist eine einfache Kontrolle des Desinfektionsprogrammes möglich und eine zusätzliche Schutzfunktion während der Dialyse gegeben.

Schließlich wird die Desinfektionsflüssigkeit einer Aufnahmeeinrichtung mit einer Katalysatorvorrichtung 6 zugeführt, welche die katalytische Zersetzung z.B. des NaOCl in die Bestandteile NaCI und 02 bewirkt. Ebenso könnten andere Desinfektionsmittel wie beispiesweise Ozon zersetzt werden.

Diese Katalysatorvorrichtung 6 kann ebenfalls ohne weiteres im Dialysegerät integriert werden. Ferner kann sie extern bzw. zentral für mehrere Geräte einer Dialysestation vorgesehen sein.

Das bei einer katalytischen Reaktion beispielsweise erzeugte NaCl kann dann über eine Leitungseinrichtung 9 dem NaOCI-Generator 3 zugeführt werden. Somit ist ein geschlossener Kreislauf zur Herstellung und katalytischen Zersetzung von NaOCI realisiert. Die Ausgangsstoffe NaCI und H2O reagieren zu NaOCI, welches nach seiner katalytischen Zersetzung in NaCI und 02 zerfällt.

Von diesen Ausgangsprodukten gehen keine Gefahren aus. Sowohl eine Verätzungsgefahr für den Bediener des Gerätes, als auch eine Gefahr für die Umwelt kann ausgeschlossen werden.

Wie erwähnt, kann das entstandene NaCl dem NaOCI-Generator wieder zugeführt werden. Die übrigen Ausgangsprodukte können ohne weiteres dem Abwasser zugeführt werden. Eine Verwendung des sauren Teildialysats bzw. - dialysatkonzentrats, in dem NaCl enthalten ist, ist ebenfalls denkbar.

Die verschiedenen Vorrichtungen können innerhalb eines Dialysegerätes angeordnet werden. Es ist somit ein integrierter Katalysator im Dialysegerät zur Verfügung gestellt, welcher wesentliche Vorteile bezüglich der Abwasserbelastung gegenüber herkömmlichen Geräten aufweist.

Wie erwähnt, kann das erfindungsgemäße Verfahren integriert in einem solchen Dialysegerät durchgeführt werden. Es ist jedoch gleichfalls möglich, wenigstens einen der Verfahrensschritte UV-Photooxidation, NaOCl-Generierung, OzonErzeugung, Bestimmung des Desinfektionsmittelgehaltes mittels Redox-Elektrode oder katalytische Zersetzung von NaOCI außerhalb der Dialysemaschine durchzuführen.

## Patentansprüche

1. Verfahren zur Desinfektion eines Dialysegerätes, wobei
die keimfrei zu haltenden Bereiche des Dialysegerätes mit einem Desinfektionsmittel desinfiziert werden **gekennzeichnet dadurch, daß**
das hierbei verwendete Desinfektionsmittel anschließend katalytisch zersetzt wird.

2. Verfahren nach Anspruch 1, **dadurch gekennzeichnet, daß** das Desinfektionsmittel NaOCI ist.

3. Verfahren nach Anspruch 2, **gekennzeichnet durch** den weiteren Verfahrensschritt, daß das in der Desinfektionsflüssigkeit verwendete NaOCI **durch** Elektrolyse einer NaCl-Lösung erzeugt wird.

4. Verfahren nach einem der Ansprüche 1 oder 3, **dadurch gekennzeichnet daß** das Desinfektionsmittel Ozon ist.

5. Verfahren nach einem der Ansprüche 1 bis 3, **gekennzeichnet durch** den weiteren Verfahrensschritt, daß das **durch** die katalytische Zersetzung erzeugte NaCI wenigstens teilweise zur elektrolytischen Erzeugung von NaOCI in einem NaOCI-Generator verwendet wird.

6. Verfahren nach einem der Ansprüche 1 bis 5, **gekennzeichnet durch** den weiteren Verfahrensschritt, daß das Vorhandensein von Desinfektionsflüssigkeit bestimmt wird.

7. Verfahren nach Anspruch 6, **dadurch gekennzeichnet, daß** die Bestimmung des Desinfektionsmittelgehaltes unmittelbar vor und/oder nach der katalytischen Zersetzung des NaOCI bestimmt wird.

8. Verfahren nach einem der Ansprüche 6 oder 7, **dadurch gekennzeichnet, daß** die Bestimmung des Desinfektionsmittels mittels einer Redox-Elektrode erfolgt.

9. Dialysevorrichtung zur Durchführung des Verfahrens nach Anspruch 1, **dadurch gekennzeichnet, daß** sie Mittel zur katalytischen Zersetzung der Desinfektionsflüssigkeit nach dem Desinfektionsvorgang aufweist.

10. Vorrichtung nach Anspruch 9, **dadurch gekennzeichnet, daß** das Desinfektionsmittel NaOCI ist.

11. Vorrichtung nach Anspruch 9, **dadurch gekennzeichnet, daß** das Desinfektionsmittel Ozon ist.

12. Vorrichtung nach Anspruch 9 oder 10 zur Durchführung des Verfahrens nach Anspruch 2, **dadurch gekennzeichnet, daß** sie einen Generator zur Herstellung von NaOCI durch Elektrolyse von NaCl aufweist.

13. Vorrichtung nach einem der Ansprüche 9 oder 12 zur Durchführung des Verfahrens nach Anspruch 3, **dadurch gekennzeichnet, daß** sie eine UV-Photooxidationskammer zur UV-Bestrahlung von RO-Wasser aufweist.

14. Vorrichtung nach einem der Ansprüche 9 bis 13 zur Durchführung des Verfahrens nach Anspruch 4, **dadurch gekennzeichnet, daß** sie wenigstens ein Mittel zur Herstellung von Ozon durch Elektrolyse aufweist.

15. Vorrichtung nach einem der Ansprüche 9 bis 14 zur Durchführung des Verfahrens nach Anspruch 5, **dadurch gekennzeichnet, daß** sie eine Leitungseinrichtung aufweist, über welche das bei der katalytischen Zersetzung erzeugte NaCl dem NaOCI-Generator wieder zugeführt wird.

16. Vorrichtung nach einem der Ansprüche 9 bis 15 zur Durchführung des Verfahrens nach einem der Ansprüche 6 bis 8, **dadurch gekennzeichnet, daß** sie eine Redox-Elektrode zum Nachweis der Anwesenheit von Desinfektionsmittel in der Dialysatleitung im Dialysegerät aufweist.

## Claims

1. Method for disinfecting a dialysis apparatus, wherein the areas of the dialysis apparatus which are to be kept sterile are disinfected with a disinfectant, **characterized in that** the disinfectant used for this purpose is then decomposed catalytically.

2. Method according to Claim 1, **characterized in that** the disinfectant is NaOCl

3. Method according to Claim 2, **characterized by** the further step that the NaOCl used in the disinfectant liquid is produced by electrolysis of an NaCl solution.

4. Method according to either of Claims 1 or 3, **characterized in that** the disinfectant is ozone.

5. Method according to one of Claims 1 to 3, **characterized by** the further step that the NaCl produced by catalytic decomposition is at least partially used for electrolytic production of NaOCl in an NaOCl generator.

6. Method according to one of Claims 1 to 5, **characterized by** the further step that the presence of disinfectant liquid is determined.

7. Method according to Claim 6, **characterized in that** the determination of the disinfectant content takes place immediately before and/or after the catalytic decomposition of the NaOCl.

8. Method according to one of Claims 6 or 7, **characterized in that** the determination of the disinfectant is carried out with a redox electrode.

9. Dialysis device for performing the method according to Claim 1, **characterized in that** it has means for catalytic decomposition of the disinfectant liquid after the disinfection process.

10. Device according to Claim 9, **characterized in that** the disinfectant is NaOCl.

11. Device according to Claim 9, **characterized in that** the disinfectant is ozone.

12. Device according to Claim 9 or 10 for performing the method according to Claim 2, **characterized in that** it has a generator for production of NaOCl by electrolysis of NaCl.

13. Device according to either of Claims 9 or 12 for performing the method according to Claim 3, **characterized in that** it has a UV photo-oxidation chamber for UV irradiation of RO water.

14. Device according to one of Claims 9 to 13 for performing the method according to Claim 4, **characterized in that** it has at least one means for production of ozone by electrolysis.

15. Device according to one of Claims 9 to 14 for performing the method according to Claim 5, **characterized in that** it has a feed unit via which the NaCl produced in the catalytic decomposition is returned to the NaOCl generator.

16. Device according to one of Claims 9 to 15 for performing the method according to one of Claims 6 to 8, **characterized in that** it has a redox electrode for detecting the presence of disinfectant in the dialysate line in the dialysis apparatus.

## Revendications

1. Procédé de désinfection d'un appareil de dialyse, dans lequel les zones à conserver dans des conditions stériles dans l'appareil de dialyse sont désinfectées au moyen d'un désinfectant, **caractérisé en ce que** le désinfectant utilisé à cet effet est décomposé ensuite par voie catalytique.

2. Procédé selon la revendication 1, **caractérisé en ce que** le désinfectant est le NaOCl.

3. Procédé selon la revendication 2, **caractérisé par** l'étape du procédé suivante, en ce que le NaOCl utilisé dans le liquide de désinfection est obtenu par l'électrolyse d'une solution de NaCl.

4. Procédé selon la revendication 1 ou 3, **caractérisé en ce que** le désinfectant est l'ozone.

5. Procédé selon l'une quelconque des revendications 1 à 3, **caractérisé par** l'étape du procédé suivante, en ce que le NaCl obtenu par décomposition catalytique est utilisé au moins en partie pour la production électrolytique du NaOCl dans un générateur de NaOCl.

6. Procédé selon l'une quelconque des revendications 1 à 5, **caractérisé par** l'étape du procédé suivante, en ce que l'on contrôle la présence du liquide de désinfection.

7. Procédé selon la revendication 6, **caractérisé en ce que** la teneur en désinfectant est déterminée directement avant et/ou après la décomposition catalytique de NaOCl.

8. Procédé selon la revendication 6 ou 7, **caractérisé en ce que** la présence de désinfectant est déterminée au moyen d'une électrode redox.

9. Dispositif de dialyse destiné à la mise en oeuvre du procédé selon la revendication 1, **caractérisé en ce que** ledit dispositif comporte des moyens pour la décomposition catalytique du liquide de désinfection après le processus de désinfection.

10. Dispositif selon la revendication 9, **caractérisé en ce que** le désinfectant est le NaOCl.

11. Dispositif selon la revendication 9, **caractérisé en ce que** le désinfectant est l'ozone.

12. Dispositif selon la revendication 9 ou 10, destiné à la mise en oeuvre du procédé selon la revendication 2, **caractérisé en ce que** ledit dispositif comporte un générateur, dans lequel le NaOCl est obtenu par l'électrolyse de NaCl.

13. Dispositif selon la revendication 9 ou 12, destiné à la mise en oeuvre du procédé selon la revendication 3, **caractérisé en ce que** ledit dispositif comporte une chambre de photo-oxydation par radiation UV, dans laquelle l'eau purifiée par osmose inverse est exposée à un rayonnement UV.

14. Dispositif selon l'une quelconque des revendications 9 à 13, destiné à la mise en oeuvre du procédé selon la revendication 4, **caractérisé en ce que** ledit dispositif comporte au moins un moyen pour la production d'ozone par électrolyse.

15. Dispositif selon l'une quelconque des revendications 9 à 14, destiné à la mise en oeuvre du procédé selon la revendication 5, **caractérisé en ce que** ledit dispositif comporte un système de conduites, par lequel le NaCl obtenu par décomposition catalytique est à nouveau acheminé vers le générateur de NaOCl.

16. Dispositif selon l'une quelconque des revendications 9 à 15, destiné à la mise en oeuvre du procédé selon l'une quelconque des revendications 6 à 8, **caractérisé en ce que** ledit dispositif comporte une électrode redox destinée à contrôler la présence de désinfectant dans la conduite du dialysat dans l'appareil de dialyse.
